# EUROPEAN PATENT SPECIFICATION

(11) **EP 3 305 728 B1**
(45) Date of publication and mention of the grant of the patent: **18.09.2019**
(21) Application number: 16799542.2
(22) Date of filing: 17.05.2016
(51) Int. Cl.: C01G 9/02, A61K 8/27, A61Q 17/00, C09C 1/30, A61K 8/25, A61K 8/89, A61K 8/02, A61Q 17/04

(54) **ZINC OXIDE-CONTAINING COMPOSITE PARTICLES, COMPOSITION FOR BLOCKING UV RAYS, AND COSMETIC MATERIAL**
ZINKOXID-HALTIGE VERBUNDSTOFFPARTIKEL, ZUSAMMENSETZUNG ZUR BLOCKIERUNG VON UV-STRAHLEN UND KOSMETISCHES MATERIAL
PARTICULES COMPOSITES CONTENANT DE L'OXYDE DE ZINC, COMPOSITION DESTINÉE À BLOQUER LES RAYONS UV, ET SUBSTANCE COSMÉTIQUE

(30) Priority: 28.05.2015 JP 2015108048
(43) Date of publication of application: 11.04.2018
(73) Proprietor: Nippon Sheet Glass Company, Limited, Tokyo 108-6321 (JP)
(72) Inventor: SHIMOKAWA, Kosei, Tokyo 108-6321 (JP); DOSHITA, Kazuhiro, Tokyo 108-6321 (JP); YAMAJI, Koichi, Osaka-shi Osaka 551-0022 (JP); YAMASHITA, Jun, Osaka-shi Osaka 551-0022 (JP)
(74) Representative: Cabinet Beau de Loménie
(86) International application number: PCT/JP2016/002408
(87) International publication number: WO 2016/189828

(56) References cited:
- WO-A1-2014/097972
- WO-A1-2014/141742
- JP-A- H0 217 932
- JP-A- 2008 094 917
- JP-A- 2014 122 306
- JP-B2- 3 503 814
- US-A1- 2006 167 138

## Description

### TECHNICAL FIELD

The present invention relates to a zinc oxide-containing composite particle, an ultraviolet-shielding composition, and a cosmetic.

### BACKGROUND ART

Zinc oxide (ZnO) has conventionally been incorporated in cosmetics, for example, as an agent for shielding against UV-A (ultraviolet rays with wavelengths from 320 nm to 400 nm). Zinc oxide, being an oxide of zinc which is an amphoteric element, is easily soluble in both acidic solutions and basic solutions and is also soluble, albeit slightly, in water which is approximately neutral. When a product such as a cosmetic contains zinc oxide, dissolution of zinc ions from zinc oxide may impair some of the properties of the product. Various techniques have been proposed to inhibit the dissolution of zinc ions from zinc oxide.

For example, Patent Literature 1 describes a composite particle for use in cosmetics, the particle including a specific polyolefin resin and zinc oxide.

Patent Literature 2 describes a zinc oxide material with a coated surface, the material including zinc oxide particles, a titania-containing coating provided on the surface of the zinc oxide particles, and a silica-containing coating provided on the titania-containing coating.

Patent Literature 3 describes a method for producing a zinc oxide material with a coated surface, the method including the steps of: obtaining zinc oxide particles by reacting a zinc compound with a neutralizer in an aqueous medium; and coating the surface of the obtained zinc oxide particles with an inorganic compound such as silica in an aqueous medium without heating the particles in a gaseous phase.

Patent Literature 4 describes a zinc oxide powder coated with water-repellent fine silica particles, the powder being obtained by dispersing a zinc oxide powder in a solution of a specific acrylic silicone resin, then adding a volatile alkaline substance and a specific alkoxysilane to the resulting dispersion, and hydrolyzing the alkoxysilane. Patent Literature 11 describes a powder comprising silica-coated zinc oxide fine particles whose surfaces are further treated with a hydrophobicity-imparting agent.

Patent Literature 5 and Patent Literature 6 describe a coated zinc oxide particle having a coating layer made of a precipitable material obtained by reaction of a carboxyvinyl polymer with a divalent or trivalent metal ion.

Patent Literature 7 describes a zinc oxide-based composite powder including a zinc oxide powder and a dipeptide that binds to zinc ions to prevent the zinc ions from dissolving into water.

Patent Literature 8 describes an ultraviolet-shielding composite particle including: a core containing zinc oxide and a first resin; and a coating film provided on the surface of the core, the coating film being made of a second resin having a composition identical to or different from the composition of the first resin. Patent Literature 13 describes a composition containing in a cosmetically acceptable medium (A) at least one organic UV filter, (B) spherical composite particles with a mean size of from 1 to 20 µm comprising a matrix and an inorganic UV filter, (C) boron nitride particles and (D) spherical porous silica particles. Patent Literature 14 describes a silica-coated metal oxide particle having high ultraviolet-screening effect, produced by coating the surface of (A) a metal oxide particle selected from zinc oxide, titanium oxide, cerium oxide and zirconium oxide with (B) 5-100 wt.% siliceous substance and converting the surface-coated metal oxide particles into secondary particles composed of agglomerated ultrafine primary particles.

Patent Literature 9 describes a silicon oxide-coated zinc oxide material including zinc oxide particles with an average particle diameter of 1 nm or more and 50 nm or less and a specific silicon oxide coating provided on the surface of the zinc oxide particles.

Although making no mention of inhibition of dissolution of zinc ions from zinc oxide, Patent Literature 10 describes silica-coated fine zinc oxide particles including fine zinc oxide particles with an average particle diameter of 5 to 100 nm as a base material, the base material being coated with silica, which amounts to 15 to 40 mass% relative to the base material.

Patent Literature 12 describes nanoparticles including a metal nanoparticle core and a shell layer based on silica.

### CITATION LIST

### Patent Literature

Patent Literature 1: JP 2006-219437 A
Patent Literature 2: JP 2008-94917 A
Patent Literature 3: JP 2008-273760 A
Patent Literature 4: JP 2009-23981 A
Patent Literature 5: JP 2011-102291 A
Patent Literature 6: JP 2012-207039 A
Patent Literature 7: JP 2014-43376 A
Patent Literature 8: JP 2014-84448 A
Patent Literature 9: JP 2015-6976 A
Patent Literature 10: JP 2007-016111 A
Patent Literature 11: US 2006/0167138 A1
Patent Literature 12: WO 2014/141742 A1
Patent Literature 13: WO 2014/097972 A1
Patent Literature 14: JP 3 503814 B2

### SUMMARY OF INVENTION

### Technical Problem

The techniques described in Patent Literature 2 and Patent Literature 3 take into account the acid resistance of zinc oxide materials with a coated surface; however, the acid resistance of composite particles containing zinc oxide has not been fully considered. There is still room for designing a zinc oxide-containing composite particle having acid resistance from a new point of view. It is therefore an object of the present invention to provide a novel zinc oxide-containing composite particle having high acid resistance.

### Solution to Problem

The present invention provides a zinc oxide-containing composite particle including: a porous silica particle; zinc oxide particles enclosed within the porous silica particle; and a coating layer covering the porous silica particle, the coating layer containing a polysilsesquioxane.

The present invention also provides an ultraviolet-shielding composition including the above zinc oxide-containing composite particle.

The present invention also provides a cosmetic including the above zinc oxide-containing composite particle.

### Advantageous Effects of Invention

The above zinc oxide-containing composite particle has high acid resistance because the porous silica particle is covered with the polysilsesquioxane-containing coating layer. It is also possible to provide an ultraviolet-shielding composition or cosmetic including the zinc oxide-containing composite particle having high acid resistance.

### BRIEF DESCRIPTION OF DRAWING

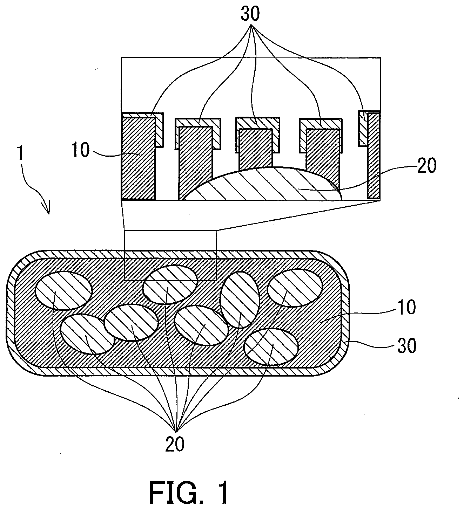

FIG. 1 is a cross-sectional view schematically illustrating the structure of a zinc oxide-containing composite particle according to an embodiment of the present invention.

### DESCRIPTION OF EMBODIMENTS

Hereinafter, an embodiment of the present invention will be described with reference to the drawings. The following description relates to examples of the present invention, and the present invention is not limited to these examples.

As shown in FIG. 1, a zinc oxide-containing composite particle 1 includes a porous silica particle 10, zinc oxide particles 20, and a coating layer 30. The zinc oxide particles 20 are enclosed within the porous silica particle 10. The coating layer 30 covers the porous silica particle 10 and contains a polysilsesquioxane.

The polysilsesquioxane contained in the coating layer 30 is formed, for example, by hydrolysis and dehydration condensation of a trifunctional alkoxysilane.

For example, when an acidic aqueous solution is present around the surface of the zinc oxide-containing composite particle 1, the coating layer 30, coupled with the porous structure of the porous silica particle 10, prevents contact of water with the zinc oxide particles 20 enclosed within the porous silica particle 10. Due to containing a polysilsesquioxane, the coating layer 30 can effectively block the penetration of water into the internal pores of the porous silica particle 10. This prevents dissolution of zinc ions from the zinc oxide particles 20, thus allowing the zinc oxide-containing composite particle 1 to have high acid resistance. Additionally, the water repellency of the coating layer 30 is adjusted by the incorporation of the polysilsesquioxane into the coating layer 30 so that the zinc oxide-containing composite particles 1 can be uniformly dispersed in an acidic aqueous solution. That is, the coating layer 30 has such a surface energy as to allow the zinc oxide-containing composite particles 1 to be uniformly dispersed in an acidic aqueous solution. Such water repellency of the coating layer 30 makes possible both prevention of contact of water with the zinc oxide particles 20 and uniform dispersion of the zinc oxide-containing composite particles 1 in an acidic aqueous solution.

The polysilsesquioxane contained in the coating layer 30 is, for example, a polysilsesquioxane in which an alkyl group having 16 or less carbon atoms is bonded to a silicon atom. This further ensures that the zinc oxide-containing composite particles 1 have high acid resistance and are uniformly dispersed in an acidic aqueous solution. The alkyl group may be linear or branched.

The polysilsesquioxane contained in the coating layer 30 desirably includes at least one polysilsesquioxane selected from the group consisting of polymethylsilsesquioxane, polyethylsilsesquioxane, polypropylsilsesquioxane, and polyoctylsilsesquioxane. Polymethylsilsesquioxane is a polysiloxane having a principal structural unit in which one methyl group is bonded to one silicon atom. Polyethylsilsesquioxane is a polysiloxane having a principal structural unit in which one ethyl group is bonded to one silicon atom. Polypropylsilsesquioxane is a polysiloxane having a principal structural unit in which one 1-propyl group or one 2-propyl group is bonded to one silicon atom. Polyoctylsilsesquioxane is a polysiloxane having a principal structural unit in which one octyl group is bonded to one silicon atom. The octyl group may be linear or branched. The inclusion of the at least one polysilsesquioxane further ensures that the zinc oxide-containing composite particles 1 have high acid resistance and are uniformly dispersed in an acidic aqueous solution.

The weight-average molecular weight of the polysilsesquioxane contained in the coating layer 30 is, for example, 500 to 100,000. The weight-average molecular weight of the polysilsesquioxane can be measured by gel-permeation chromatography (GPC) using standard polystyrene as a standard polymer.

The mass of the coating layer 30 is, for example, 10% to 80% of the mass of the zinc oxide-containing composite particle 1. In this case, the coating layer 30 can more effectively protect the porous silica particle, further ensuring that the zinc oxide-containing composite particles 1 have high acid resistance and are uniformly dispersed in an acidic aqueous solution.

The shape of the porous silica particle 10 is not particularly limited. For example, the porous silica particle 10 is flaky. The term "flaky" as used herein means having the shape of a plate whose principal surfaces can be considered flat or curved and have a diameter the ratio of which to the thickness of the plate is 2 or more. The diameter of a principal surface refers to the diameter of a circle having an area equal to the area of the principal surface. When the porous silica particle 10 is flaky, the largest diameter of the principal surfaces of the porous silica particle 10 is, for example, 5 µm to 100 µm, and the thickness of the porous silica particle 10 is, for example, 0.2 µm to 2 µm. With the use of the flaky porous silica particles 10, a product containing the zinc oxide-containing composite particles 1 can be provided with desired properties appropriate for the intended use of the product.

The porous silica particle 10 may be spherical. The term "spherical" as used herein means that the porous silica particle 10 has the shape of a granule in which the ratio of its largest diameter to its smallest diameter is 1 to 1.5. When the spherical porous silica particles 10 are used, the average particle diameter of the porous silica particles 10 is, for example, 1 µm to 100 µm. With the use of the spherical porous silica particles 10, a product containing the zinc oxide-containing composite particles 1 can be provided with desired properties appropriate for the intended use of the product. The average particle diameter of the porous silica particles 10, as defined herein, refers to a particle diameter at a cumulative volume of 50% in a particle size distribution measured with a laser diffraction particle size analyzer.

The porous silica particle 10 has, for example, a mesoporous structure having mesopores with diameters of 2 to 50 nm. The pore size distribution of the porous silica particle 10, as measured by a nitrogen adsorption method, desirably has a peak in the range of 2 nm to 50 nm. In this case, the zinc oxide particles 20 can be properly held within the porous silica particle 10, and penetration of water into the porous silica particle 10 can be effectively blocked.

The average particle diameter (average primary particle diameter) of the zinc oxide particles 20 is not particularly limited, as long as it is smaller than the smallest diameter or thickness of the porous silica particle 10. For example, the average particle diameter of the zinc oxide particles 20 is 10 nm to 1000 nm. In this case, the zinc oxide particles 20 are more likely to be properly held within the porous silica particle 10. Thus, when an acidic aqueous solution is present around the surface of the zinc oxide-containing composite particle 1, the dissolution of zinc ions from the zinc oxide particles 20 is inhibited. The average particle diameter of the zinc oxide particles, as defined herein, refers to a particle diameter at a cumulative volume of 50% in a particle size distribution measured with a laser diffraction particle size analyzer.

Zinc oxide acts as a UV-A shielding agent; therefore, the use of the zinc oxide-containing composite particles 1 makes it possible to provide an ultraviolet-shielding composition containing the zinc oxide-containing composite particles 1.

The use of the zinc oxide-containing composite particles 1 also makes it possible to provide a cosmetic containing the zinc oxide-containing composite particles 1. This cosmetic has a UV-A shielding property due to containing the zinc oxide-containing composite particles 1. The cosmetic is, for example, an aqueous cosmetic of the O/W type containing a carboxyvinyl polymer. In this case, the viscosity of the cosmetic can be adjusted to a desired range by the presence of the carboxyvinyl polymer.

For example, if zinc oxide is incorporated into an aqueous cosmetic of the O/W type whose aqueous phase contains a carboxyvinyl polymer as a viscosity modifier, dissolved zinc ions may interact with the carboxyvinyl polymer to decrease the viscosity of the system. This is why the incorporation of zinc oxide into aqueous cosmetics is not feasible in many cases. However, the zinc oxide-containing composite particle 1 has high acid resistance. Thus, when the zinc oxide-containing composite particles 1 are incorporated into an aqueous cosmetic of the O/W type containing a carboxyvinyl polymer, dissolution of zinc ions from the zinc oxide particles 20 and hence interaction of zinc ions with the carboxyvinyl polymer are prevented. The use of the zinc oxide-containing composite particles 1 in a cosmetic therefore allows both a carboxyvinyl polymer as a viscosity modifier and zinc oxide to be present in the cosmetic in a preferred manner without dissolution of zinc ions. The zinc oxide-containing composite particles 1 can, as described above, be incorporated into an aqueous cosmetic of the O/W type containing a carboxyvinyl polymer, and this incorporation of the zinc oxide-containing composite particles 1 enables the cosmetic to have a desired viscosity and also to maintain the UV-A shielding property over a long period of time.

Any of the following substances may be deposited or adsorbed on the surface of the zinc oxide-containing composite particles according to the present invention: amphiphilic materials such as a surfactant; and hydrophilic polymers such as a silane compound having a polyether group, polyvinyl alcohol, polyethylene glycol, and polyvinylpyrrolidone. In this case, the dispersibility of the zinc oxide-containing composite particles in water is adjusted.

The following describes an exemplary method for producing the zinc oxide-containing composite particles 1. First, an exemplary method for making flaky porous silica particles each enclosing zinc oxide particles therein will be described. A silica sol containing colloidal silica particles as a dispersed phase and water as a dispersion medium is prepared. The pH of the silica sol is, for example, 7 or more. The average primary particle diameter of the colloidal silica particles in the silica sol is, for example, 5 nm to 500 nm. Zinc oxide particles are added and dispersed in the silica sol. To this end, a powder of zinc oxide particles may be directly added to the silica sol; however, it is desirable to prepare a dispersion of zinc oxide particles using, for example, a wet bead mill, and mix and stir the dispersion and the silica sol to disperse the zinc oxide particles in the silica sol. Next, the silica sol containing the dispersed zinc oxide particles is introduced into a liquid containing a specific organic solvent. The organic solvent used can be, for example, a protic solvent having a relative permittivity of 30 or less at 20°C and being miscible with water or an aprotic solvent having a relative permittivity of 40 or less at 20°C and being miscible with water. For example, the silica sol containing the dispersed zinc oxide particles is introduced dropwise into the liquid containing the specific organic solvent. The liquid containing the specific organic solvent is desirably stirred during the introduction of the silica sol containing the dispersed zinc oxide particles. The introduction of the silica sol results in formation of flaky aggregates of the colloidal silica particles in the liquid containing the specific organic solvent. The zinc oxide particles are incorporated and dispersed within the aggregates.

Next, the aggregates are separated from the liquid containing the specific organic solvent. The separation of the aggregates can be accomplished by a known technique such as centrifugation, filtration, or decantation. The separated aggregates are washed and dried if necessary. For example, the aggregates are dried in an atmosphere with a temperature of 40°C to 250°C, desirably 50°C to 200°C. The drying causes gelation of the aggregates. This enhances the binding strength between the colloidal silica particles constituting the flaky aggregates produced. The gelled aggregates are heat-treated if necessary to remove unwanted organic substances or control the pores. This heat treatment is carried out, for example, at 300°C to 800°C. The heat treatment prevents the decrease in the crystallinity of zinc oxide caused by reaction between silica and zinc oxide and also allows the retention of the UV-A shielding property of zinc oxide. If the temperature of the heat treatment of the aggregates is lower than 300°C, the heat treatment cannot be very effective, while if the temperature of the heat treatment of the aggregates is higher than 800°C, the UV-A shielding property of zinc oxide can be deteriorated. It is desirable to carry out the heat treatment of the aggregates at 400°C to 600°C to make the heat treatment more effective and achieve better retention of the UV-A shielding property of zinc oxide. In the manner described above, flaky porous silica particles each enclosing zinc oxide particles therein are obtained.

Alternatively, the silica sol containing the dispersed zinc oxide particles may be introduced into a specific aqueous electrolyte solution rather than into a liquid containing a specific organic solvent. In this case, the aqueous electrolyte solution is desirably stirred during the introduction of the silica sol containing the dispersed zinc oxide particles. The aqueous electrolyte solution used can be an aqueous solution prepared by adding 0.3 parts by weight or more of an electrolyte to 100 parts by weight of water. The aqueous electrolyte solution used can be, for example, an aqueous solution of at least one electrolyte selected from NaCl, CaCl₂, CH₃COONa, NaNO₃, KCl, (CH₃COO)₂Mg•4H₂O, and KNO₃. The introduction of the silica sol results in formation of flaky aggregates of the colloidal silica particles in the aqueous electrolyte solution. The zinc oxide particles are incorporated and dispersed within the aggregates. It is desirable to heat the aqueous electrolyte solution after the formation of the aggregates but before separation of the aggregates from the aqueous electrolyte solution. For example, the aqueous electrolyte solution is heated to boiling. This enhances the binding strength between the colloidal silica particles constituting the aggregates. Also in this manner, flaky porous silica particles each enclosing zinc oxide particles therein can be obtained. The aggregates are heat-treated if necessary. The heat treatment of the aggregates is carried out, for example, at 300°C to 800°C, desirably at 400°C to 600°C, to prevent the decrease in the crystallinity of zinc oxide caused by reaction between silica and zinc oxide and allow the retention of the UV-A shielding property of zinc oxide.

Next, an exemplary method for making spherical porous silica particles each enclosing zinc oxide particles therein will be described. First, a silica sol containing colloidal silica particles as a dispersed phase and water as a dispersion medium is prepared. The average primary particle diameter of the colloidal silica particles in the silica sol is, for example, 5 nm to 500 nm. Zinc oxide particles are added and dispersed in the silica sol. To this end, a powder of zinc oxide particles may be directly added to the silica sol; however, it is desirable to prepare a dispersion of zinc oxide particles using, for example, a wet bead mill, and mix and stir the dispersion and the silica sol to disperse the zinc oxide particles in the silica sol. Next, the silica sol containing the dispersed zinc oxide particles is spray-dried. This results in spherical porous silica particles each enclosing zinc oxide particles therein. The temperature of a drying oven used in the spray drying is, for example, 50°C to 200°C. The conditions of the spraying are adjusted so that, for example, the resulting spherical porous silica particles will have an average particle diameter of 1 µm to 100 µm. The particles resulting from the spray drying are heat-treated at 300°C to 800°C if necessary. The heat treatment of the particles resulting from the spray drying is desirably carried out at 400°C to 600°C to prevent the decrease in the crystallinity of zinc oxide caused by reaction between silica and zinc oxide, allow the retention of the UV-A shielding property of zinc oxide, and achieve effective removal of organic substances or effective control of the pores.

The thus-obtained flaky or spherical porous silica particles each enclosing zinc oxide particles therein are added to a solution containing a polysilsesquioxane or a solution prepared by hydrolysis and dehydration condensation of a trifunctional alkoxysilane. Next, the solution is heated under stirring to remove the solvent from the solution. As a result, the porous silica particles are covered with a polysilsesquioxane-containing coating layer, and thus zinc oxide-containing composite particles according to the present invention are produced.

### EXAMPLES

The present invention will be described in more detail with examples. The present invention is not limited to the examples presented below.

### <Example 1>

To 67.5 parts by mass of pure water were added 30 parts by mass of zinc oxide particles (manufactured by Tayca Corporation under the product name "MZ-500") and 2.5 parts by mass of a special polycarboxylate polymer-type surfactant (manufactured by Kao Corporation under the product name "POIZ 532A"), and the mixture was stirred and circulated together with 4.3 kg of 0.65-mm-diameter zirconia beads by a horizontal, continuous-type wet-grinding mill (manufactured by Shinmaru Enterprises Corporation under the product name "DYNO-MILL KDL-PILOT A") for 3 hours (stirring speed: peripheral speed = 10 m/sec, flow speed = 5 L/min), and thus a dispersion of zinc oxide particles (diameter of dispersed zinc oxide particles = about 0.2 µm) was obtained.

Next, 20 parts by mass of the dispersion of zinc oxide particles was mixed with 30 parts by mass of a silica sol (manufactured by Nippon Chemical Industrial Co., Ltd. under the product name "SILICADOL 30S" and containing water as a dispersion medium and silica particles having an average primary particle diameter of 9 nm) to obtain a sol A. The content of zinc oxide particles in solids of the sol A was 20 mass%. Next, 100 ml of 2-propanol was placed in a beaker, and 1 g drops of the sol A were added one by one to 2-propanol stirred in the beaker. The dropwise addition of the sol A produced flaky aggregates.

The aggregates were separated from 2-propanol by decantation, the separated aggregates were dried in a vacuum dryer at 120°C, and the dried aggregates were heat-treated at 500°C for 7 hours. As a result, flaky porous silica particles B1 each enclosing zinc oxide particles therein were obtained (average thickness = 0.8 µm, average particle diameter = 20 µm, zinc oxide content = 20 mass%). The average particle diameter mentioned above is a particle diameter (D50) at a cumulative volume of 50% in a particle size distribution measured with a laser diffraction particle size analyzer. The pore size distribution of the porous silica particles B1 was measured by a nitrogen adsorption method, and the porous silica particles B1 were found to have a pore size distribution with a peak at 20 nm.

The porous silica particles B1 were added to an ethanol solution of 20 mass% polymethylsilsesquioxane (manufactured by Konishi Chemical Ind. Co., Ltd. under the product name "SR-13"), and this solution was heated under stirring to remove ethanol contained as the solvent. Afterwards, the porous silica particles B1 coated with polymethylsilsesquioxane were collected. In this manner, zinc oxide-containing composite particles according to Example 1 were obtained. The mass of the coating layer of polymethylsilsesquioxane in each of the zinc oxide-containing composite particles according to Example 1 was 20% of the overall mass of the zinc oxide-containing composite particle.

The zinc oxide-containing composite particles according to Example 1 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 1 were uniformly dispersed in the aqueous citric acid solution. The pH of the aqueous solution was about 3 at 1 hour after the dispersing of the zinc oxide-containing composite particles according to Example 1 and, thereafter, the pH of the aqueous solution remained almost unchanged. This leads to the conclusion that zinc ions hardly dissolved from zinc oxide enclosed within the porous silica particles of the zinc oxide-containing composite particles according to Example 1.

### <Example 2>

An amount of 65.9 g of isopropyl alcohol as a solvent, 29.5 g of methyltriethoxysilane (manufactured by Evonik Industries under the product name "Dynasylan MTES"), and 4.6 g of a 0.01 N nitric acid containing water for hydrolysis and serving as a hydrolysis catalyst were mixed. The mixture was stirred at a constant temperature of 30°C for 1 hour, and thus hydrolysis and dehydration condensation of methyltriethoxysilane were allowed to proceed to give a coating liquid C. The porous silica particles B1 were added to the coating liquid C, and the coating liquid C was heated under stirring to remove the solvent from the coating liquid C. Afterwards, the porous silica particles B1 coated with polymethylsilsesquioxane resulting from the hydrolysis and dehydration condensation of methyltriethoxysilane were collected. In this manner, zinc oxide-containing composite particles according to Example 2 were obtained. The mass of the coating layer of polymethylsilsesquioxane in each of the zinc oxide-containing composite particles according to Example 2 was 10% of the overall mass of the zinc oxide-containing composite particle.

The zinc oxide-containing composite particles according to Example 2 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 2 were uniformly dispersed in the aqueous citric acid solution. The pH of the aqueous solution was about 3 at 1 hour after the dispersing of the zinc oxide-containing composite particles according to Example 2 and, thereafter, the pH of the aqueous solution remained almost unchanged.

### <Example 3>

The sol A was spray-dried using a spray dryer (manufactured by Fujisaki Electric Co., Ltd. under the product name "MDL-050L"). The temperature of the drying oven in the spray drying was adjusted to about 65°C. In this manner, spherical porous silica particles B2 each enclosing zinc oxide particles therein were obtained (average particle diameter = 5 µm, zinc oxide content = about 20 mass%). The average particle diameter mentioned above is a particle diameter (D50) at a cumulative volume of 50% in a particle size distribution measured with a laser diffraction particle size analyzer. The pore size distribution of the porous silica particles B2 was measured by a nitrogen adsorption method, and the porous silica particles B2 were found to have a pore size distribution with a peak at around 3 nm.

The porous silica particles B2 were added to an ethanol solution of 20 mass% polymethylsilsesquioxane (manufactured by Konishi Chemical Ind. Co., Ltd. under the product name "SR-13"), and this solution was heated under stirring to remove ethanol contained as the solvent. Afterwards, the porous silica particles B2 coated with polymethylsilsesquioxane were collected. In this manner, zinc oxide-containing composite particles according to Example 3 were obtained. The mass of the coating layer of polymethylsilsesquioxane in each of the zinc oxide-containing composite particles according to Example 3 was 20% of the overall mass of the zinc oxide-containing composite particle.

The zinc oxide-containing composite particles according to Example 3 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 3 were uniformly dispersed in the aqueous citric acid solution. The pH of the aqueous solution was about 3 at 1 hour after the dispersing of the zinc oxide-containing composite particles according to Example 3 and, thereafter, the pH of the aqueous solution remained almost unchanged.

### <Example 4>

An amount of 65.9 g of isopropyl alcohol as a solvent, 29.5 g of methyltriethoxysilane (manufactured by Evonik Industries under the product name "Dynasylan MTES"), and 4.6 g of a 0.01 N nitric acid containing water for hydrolysis and serving as a hydrolysis catalyst were mixed. The mixture was stirred at a constant temperature of 30°C for 1 hour, and thus hydrolysis and dehydration condensation of methyltriethoxysilane were allowed to proceed to give a coating liquid D. The porous silica particles B2 were added to the coating liquid D, and the coating liquid D was heated under stirring to remove the solvent from the coating liquid D. Afterwards, the porous silica particles B2 coated with polymethylsilsesquioxane resulting from the hydrolysis and dehydration condensation of methyltriethoxysilane were collected. In this manner, zinc oxide-containing composite particles according to Example 4 were fabricated. The mass of the coating layer of polymethylsilsesquioxane in each of the zinc oxide-containing composite particles according to Example 4 was 10% of the overall mass of the zinc oxide-containing composite particle.

The zinc oxide-containing composite particles according to Example 4 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 4 were uniformly dispersed in the aqueous citric acid solution. The pH of the aqueous solution was about 3 at 1 hour after the dispersing of the zinc oxide-containing composite particles according to Example 4 and, thereafter, the pH of the aqueous solution remained almost unchanged.

### <Example 5>

An amount of 69.9 g of isopropyl alcohol as a solvent, 26.3 g of ethyltriethoxysilane (manufactured by Tokyo Chemical Industry Co., Ltd.), and 3.8 g of a 0.01 N nitric acid containing water for hydrolysis and serving as a hydrolysis catalyst were mixed. The mixture was stirred at a constant temperature of 30°C for 1 hour, and thus hydrolysis and dehydration condensation of ethyltriethoxysilane were allowed to proceed to give a coating liquid E. Zinc oxide-containing composite particles according to Example 5 were fabricated in the same manner as in Example 4, except for using the coating liquid E instead of the coating liquid D. The mass of the coating layer of polyethylsilsesquioxane in each of the zinc oxide-containing composite particles according to Example 5 was 10% of the overall mass of the zinc oxide-containing composite particle.

The zinc oxide-containing composite particles according to Example 5 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 5 were uniformly dispersed in the aqueous citric acid solution. The pH of the aqueous solution was about 3 at 1 hour after the dispersing of the zinc oxide-containing composite particles according to Example 5 and, thereafter, the pH of the aqueous solution remained almost unchanged.

### <Example 6>

An amount of 72.7 g of isopropyl alcohol as a solvent, 24.1 g of propyltriethoxysilane (manufactured by Evonik Industries under the product name "Dynasylan PTEO"), and 3.2 g of a 0.01 N nitric acid containing water for hydrolysis and serving as a hydrolysis catalyst were mixed. The mixture was stirred at a constant temperature of 30°C for 1 hour, and thus hydrolysis and dehydration condensation of propyltriethoxysilane were allowed to proceed to give a coating liquid F. Zinc oxide-containing composite particles according to Example 6 were fabricated in the same manner as in Example 4, except for using the coating liquid F instead of the coating liquid D. The mass of the coating layer of polypropylsilsesquioxane in each of the zinc oxide-containing composite particles according to Example 6 was 10% of the overall mass of the zinc oxide-containing composite particle.

The zinc oxide-containing composite particles according to Example 6 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles in the solution. The zinc oxide-containing composite particles according to Example 6 were uniformly dispersed in the aqueous citric acid solution. The pH of the aqueous solution was about 3 at 1 hour after the dispersing of the zinc oxide-containing composite particles according to Example 6 and, thereafter, the pH of the aqueous solution remained almost unchanged.

### <Example 7>

An amount of 79.6 g of isopropyl alcohol as a solvent, 18.6 g of octyltriethoxysilane (manufactured by Evonik Industries under the product name "Dynasylan OCTEO"), and 1.9 g of a 0.01 N nitric acid containing water for hydrolysis and serving as a hydrolysis catalyst were mixed. The mixture was stirred at a constant temperature of 30°C for 1 hour, and thus hydrolysis and dehydration condensation of octyltriethoxysilane were allowed to proceed to give a coating liquid G. Zinc oxide-containing composite particles according to Example 7 were fabricated in the same manner as in Example 4, except for using the coating liquid G instead of the coating liquid D. The mass of the coating layer of polyoctylsilsesquioxane in each of the zinc oxide-containing composite particles according to Example 7 was 10% of the overall mass of the zinc oxide-containing composite particle.

The zinc oxide-containing composite particles according to Example 7 were added to an aqueous citric acid solution with a pH of 2.5 so that the concentration of the zinc oxide-containing composite particles would be 10 mass%, and the aqueous citric acid solution was then stirred to disperse the zinc oxide-containing composite particles according to Example 7 in the solution. The zinc oxide-containing composite particles according to Example 7 were uniformly dispersed in the aqueous citric acid solution. The pH of the aqueous solution was about 3 at 1 hour after the dispersing of the zinc oxide-containing composite particles according to Example 7 and, thereafter, the pH of the aqueous solution remained almost unchanged.

### <Comparative Example 1>

The porous silica particles B1 having no coating layer were added to an aqueous citric acid solution with a pH of 2.5 so that the concentration of the porous silica particles B1 would be 10 mass%, and then the aqueous citric acid solution was stirred. The pH of the aqueous solution changed to 6 or more within 1 minute after the addition of the porous silica particles B1 having no coating layer to the aqueous citric acid solution. This leads to the conclusion that zinc ions dissolved from zinc oxide.

### <Comparative Example 2>

The porous silica particles B2 having no coating layer were added to an aqueous citric acid solution with a pH of 2.5 so that the concentration of the porous silica particles B2 would be 10 mass%, and then the aqueous citric acid solution was stirred. The pH of the aqueous solution changed to 6 or more within 1 minute after the addition of the porous silica particles B2 having no coating layer to the aqueous citric acid solution.

### <Comparative Example 3>

Zinc oxide (manufactured by Tayca Corporation under the product name "MZ-500") was added to an aqueous citric acid solution with a pH of 2.5 so that the concentration of the zinc oxide would be 10 mass%, and then the aqueous citric acid solution was stirred. The pH of the aqueous solution changed to 6 or more within 1 minute after the addition of the zinc oxide to the aqueous citric acid solution.

### <Comparative Example 4>

Zinc oxide treated with octyltriethoxysilane (manufactured by Tayca Corporation under the product name "MZX-5080TS") was added to an aqueous citric acid solution with a pH of 2.5. The zinc oxide floated on the surface of the aqueous citric acid solution, and was not dispersed in the aqueous citric acid solution even by vigorous stirring.

## Claims

1. A zinc oxide-containing composite particle (1) comprising:
a porous silica particle (10);
zinc oxide particles (20) enclosed within the porous silica particle (10); and
a coating layer (30) covering the porous silica particle, the coating layer containing a polysilsesquioxane.

2. The zinc oxide-containing composite particle (1) according to claim 1, wherein the polysilsesquioxane is formed by hydrolysis and dehydration condensation of a trifunctional alkoxysilane.

3. The zinc oxide-containing composite particle (1) according to claim 1, wherein the polysilsesquioxane is a polysilsesquioxane in which an alkyl group having 16 or less carbon atoms is bonded to a silicon atom.

4. The zinc oxide-containing composite particle (1) according to claim 1, wherein the polysilsesquioxane comprises at least one polysilsesquioxane selected from the group consisting of polymethylsilsesquioxane, polyethylsilsesquioxane, polypropylsilsesquioxane, and polyoctylsilsesquioxane.

5. The zinc oxide-containing composite particle (1) according to claim 1, wherein the porous silica particle (10) is flaky.

6. The zinc oxide-containing composite particle (1) according to claim 1, wherein the porous silica particle (10) is spherical.

7. The zinc oxide-containing composite particle (1) according to claim 1, wherein a pore size distribution of the porous silica particle (10), as measured by a nitrogen adsorption method, has a peak in the range of 2 nm to 50 nm.

8. The zinc oxide-containing composite particle (1) according to claim 1, wherein the zinc oxide particles (20) have an average particle diameter of 10 nm to 1000 nm.

9. An ultraviolet-shielding composition comprising the zinc oxide-containing composite particle (1) according to claim 1.

10. A cosmetic comprising the zinc oxide-containing composite particle according to claim 1.

11. Ultraviolet-shielding composition according to claim 9, or cosmetic according to claim 10, comprising a carboxyvinyl polymer.

12. Ultraviolet-shielding or cosmetic according to claim 11, in the form of an O/W type composition whose aqueous phase contains said carboxyvinyl polymer.

## Patentansprüche

1. Zinkoxid enthaltendes Verbundstoffteilchen (1), umfassend:
ein poröses Siliciumdioxidteilchen (10),
Zinkoxidteilchen (20), die innerhalb des porösen Siliciumdioxidteilchens (10) eingeschlossen sind, und
eine Beschichtungsschicht (30), die das poröse Siliciumdioxidteilchen bedeckt, wobei die Beschichtungsschicht ein Polysilsesquioxan enthält.

2. Zinkoxid enthaltendes Verbundstoffteilchen (1) nach Anspruch 1, wobei das Polysilsesquioxan durch Hydrolyse und Dehydratisierungskondensation eines trifunktionellen Alkoxysilans gebildet wird.

3. Zinkoxid enthaltendes Verbundstoffteilchen (1) nach Anspruch 1, wobei das Polysilsesquioxan ein Polysilsesquioxan ist, wobei eine Alkylgruppe, die 16 oder weniger Kohlenstoffatome aufweist, an ein Siliciumatom gebunden ist.

4. Zinkoxid enthaltendes Verbundstoffteilchen (1) nach Anspruch 1, wobei das Polysilsesquioxan mindestens ein Polysilsesquioxan umfasst ausgewählt aus der Gruppe bestehend aus Polymethylsilsesquioxan, Polyethylsilsesquioxan, Polypropylsilsesquioxan und Polyoctylsilsesquioxan.

5. Zinkoxid enthaltendes Verbundstoffteilchen (1) nach Anspruch 1, wobei das poröse Siliciumdioxidteilchen (10) schuppenförmig ist.

6. Zinkoxid enthaltendes Verbundstoffteilchen (1) nach Anspruch 1, wobei das poröse Siliciumdioxidteilchen (10) kugelförmig ist.

7. Zinkoxid enthaltendes Verbundstoffteilchen (1) nach Anspruch 1, wobei eine Porengrößenverteilung des porösen Siliciumdioxidteilchens (10), wie durch ein Stickstoffadsorptionsverfahren gemessen, einen Peak im Bereich von 2 nm bis 50 nm aufweist.

8. Zinkoxid enthaltendes Verbundstoffteilchen (1) nach Anspruch 1, wobei die Zinkoxidteilchen (20) einen durchschnittlichen Teilchendurchmesser von 10 nm bis 1000 nm aufweisen.

9. Ultraviolett abschirmende Zusammensetzung umfassend das Zinkoxid enthaltende Verbundstoffteilchen (1) nach Anspruch 1.

10. Kosmetikum umfassend das Zinkoxid enthaltende Verbundstoffteilchen nach Anspruch 1.

11. Ultraviolett abschirmende Zusammensetzung nach Anspruch 9 oder Kosmetikum nach Anspruch 10, umfassend ein Carboxyvinylpolymer.

12. Ultraviolett abschirmende Zusammensetzung oder Kosmetikum nach Anspruch 11 in Form einer Zusammensetzung vom Ö/W-Typ, deren wässrige Phase das Carboxyvinylpolymer enthält.

## Revendications

1. Particule composite contenant de l'oxyde de zinc (1) comprenant :
une particule de silice poreuse (10) ;
des particules d'oxyde de zinc (20) enfermées dans la particule de silice poreuse (10) ; et
une couche de revêtement (30) couvrant la particule de silice poreuse, la couche de revêtement contenant un polysilsesquioxane.

2. Particule composite contenant de l'oxyde de zinc (1) selon la revendication 1, dans laquelle le polysilsesquioxane est formé par hydrolyse et déshydration condensation d'un alcoxysilane trifonctionnel.

3. Particule composite contenant de l'oxyde de zinc (1) selon la revendication 1, dans lequel le polysilsesquioxane est un polysilsesquioxane dans lequel un groupe alkyle ayant 16 atomes de carbone ou moins est lié à un atome de silicium.

4. Particule composite contenant de l'oxyde de zinc (1) selon la revendication 1, dans lequel le polysilsesquioxane comprend au moins un polysilsesquioxane choisi dans le groupe consistant en polyméthylsilsequioxane, polyéthylsilsequioxane, polypropylsilsequioxane, et polyoctylsilsesquioxane.

5. Particule composite contenant de l'oxyde de zinc (1) selon la revendication 1, dans laquelle la particule de silice poreuse (10) est lamellaire.

6. Particule composite contenant de l'oxyde de zinc (1) selon la revendication 1, dans laquelle la particule de silice poreuse (10) est sphérique.

7. Particule composite contenant de l'oxyde de zinc (1) selon la revendication 1, dans laquelle une distribution de taille de pore de la particule de silice poreuse (10), comme mesurée par un procédé d'adsorption d'azote, présente un pic dans l'intervalle de 2 nm à 50 nm.

8. Particule composite contenant de l'oxyde de zinc (1) selon la revendication 1, dans laquelle les particules d'oxyde de zinc (20) présentent un diamètre moyen de particule de 10 nm à 1 000 nm.

9. Composition d'écran aux ultraviolets comprenant la particule composite contenant de l'oxyde de zinc (1) selon la revendication 1.

10. Cosmétique comprenant la particule composite contenant de l'oxyde de zinc selon la revendication 1.

11. Composition d'écran aux ultraviolets selon la revendication 9, ou cosmétique selon la revendication 10, comprenant un polymère de carboxyvinyle.

12. Ecran aux ultraviolets ou cosmétique selon la revendication 11, dans la forme d'une composition de type Huile/Eau dont la phase aqueuse contient ledit polymère de carboxyvinyle.
